(19) Europäisches Patentamt
European Patent Office
Office européen des brevets

(11) Veröffentlichungsnummer: **0 435 104 A2**

(12) **EUROPÄISCHE PATENTANMELDUNG**

(21) Anmeldenummer: **90124171.1**

(51) Int. Cl.[5]: **C07D 239/60**, A01N 43/54

(22) Anmeldetag: **14.12.90**

(30) Priorität: **22.12.89 DE 3942476**

(43) Veröffentlichungstag der Anmeldung: **03.07.91 Patentblatt 91/27**

(84) Benannte Vertragsstaaten: **BE CH DE ES FR GB IT LI NL**

(71) Anmelder: **BASF Aktiengesellschaft**
**Carl-Bosch-Strasse 38**
**W-6700 Ludwigshafen(DE)**

(72) Erfinder: **Eicken, Karl, Dr.**
**Am Huettenwingert 12**
**W-6706 Wachenheim(DE)**
Erfinder: **Rheinheimer, Joachim, Dr.**
**Merziger Strasse 24**
**W-6700 Ludwigshafen(DE)**
Erfinder: **Vogelbacher, Uwe Josef, Dr.**
**Niedererdstrasse 56**
**W-6700 Ludwigshafen(DE)**
Erfinder: **Westphalen, Karl-Otto, Dr.**
**Mausbergweg 58**
**W-6720 Speyer(DE)**
Erfinder: **Wuerzer, Bruno, Dr.**
**Ruedigerstrasse 13**
**W-6701 Otterstadt(DE)**
Erfinder: **Rademacher, Wilhelm, Dr.**
**Austrasse 1**
**W-6703 Limburgerhof(DE)**
Erfinder: **Grossmann, Klaus, Dr.**
**Wilhelm-Busch-Strasse 5**
**W-6703 Limburgerhof(DE)**

(54) **Salicylsäurederivate, Verfahren zu ihrer Herstellung und ihre Verwendung als Herbizide und Bioregulatoren.**

(57) Salicylsäurederivate und der Formel I,

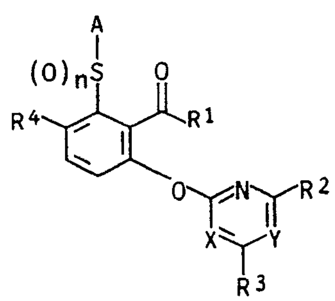

I

in der A für einen gegebenenfalls substituierten Phenylrest, einen gegebenenfalls substituierten 5-gliedrigen Heteroaromaten mit zwei bis vier Stickstoffatomen oder mit zwei Stickstoffatomen und zusätzlich einem Schwefel- oder Sauerstoffatom oder einen gegebenenfalls substituierten Naphthylrest steht, n 0, 1 oder 2 bedeutet, X und Y ein Stickstoffatom oder eine Methingruppe darstellen und die Reste $R^1$ bis $R^4$ die in der Beschreibung genannte Bedeutung haben, Verfahren und Zwischenprodukte zu ihrer Herstellung und ihre Verwendung als Herbizide und Bioregulatoren.

EP 0 435 104 A2

# SALICYLSÄUREDERIVATE, VERFAHREN ZU IHRER HERSTELLUNG UND IHRE VERWENDUNG ALS HERBIZIDE UND BIOREGULATOREN

Die vorliegende Erfindung betrifft Salicylsäurederivate der Formel I,

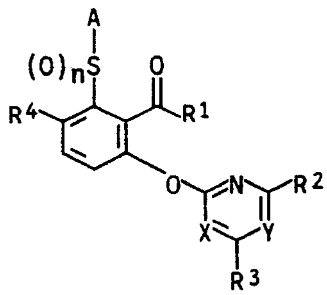

I

in der die Substituenten folgende Bedeutung haben:

$R^1$

eine Succinyliminooxygruppe;

ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

einen Rest $-OR^5$, worin

$R^5$

Wasserstoff, ein Alkalimetallkation, das Äquivalent eines Erdalkalimetallkations oder ein organisches Ammoniumion;

eine $C_3$-$C_{12}$-Cycloalkylgruppe, welche ein bis drei $C_1$-$C_4$-Alkylreste tragen kann;

eine $C_1$-$C_{10}$-Alkylgruppe, welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, $C_3$-$C_{12}$-Cycloalkyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

eine $C_1$-$C_{10}$-Alkylgruppe, welche ein bis fünf Halogenatome tragen kann und einen der folgenden Reste trägt: ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

eine $C_2$-$C_6$-Alkylgruppe, welche in der 2-Position einen der folgenden Reste trägt: $C_1$-$C_6$-Alkoxyimino, $C_3$-$C_6$-Alkenyloxyimino, $C_3$-$C_6$-Halogenalkenyloxyimino oder Benzyloxyimino;

eine $C_3$-$C_6$-Alkenyl- oder eine $C_3$-$C_6$-Alkinylgruppe, wobei diese Gruppen ihrerseits ein bis fünf Halogenatome tragen können;

unsubstituiertes oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiertes oder ein- bis fünffach durch Halogen substituiertes Phenyl;

bedeutet, oder

einen Rest $ON=CR^6R^7$, worin

$R^6$ und $R^7$

$C_1$-$C_{20}$-Alkyl, welches einen Phenylrest, eine $C_1$-$C_4$-Alkoxy- und/oder eine $C_1$-$C_4$-Alkylthiogruppe tragen kann, Phenyl oder gemeinsam eine $C_3$-$C_{12}$-Alkylenkette, welche ein bis drei $C_1$-$C_3$-Alkylgruppen tragen kann

bedeuten;

$R^2$, $R^3$

$C_1$-$C_4$-Alkyl, Halogen, $C_1$-$C_4$-Halogenalkyl, $C_1$-$C_4$-Alkoxy, $C_1$-$C_4$-Halogenalkoxy, $C_1$-$C_4$-Alkylthio und/oder Motto- oder Di-$C_1$-$C_4$-alkylamino;

n

Null, 1 oder 2;

X,Y
ein Stickstoffatom oder eine Methingruppe =CH-;

$R^4$
Wasserstoff oder $C_1$-$C_4$-Alkyl;

A
einen gegebenenfalls ein bis dreifach substituierten, im Fall von Halogen als Substituent ein bis fünffach substituierten Phenylrest

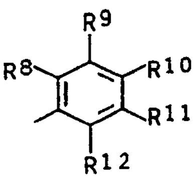

worin $R^8$ - $R^{12}$
Wasserstoff, Halogen, Cyano, Nitro;
eine $C_3$-$C_6$-Alkenyl-, $C_3$-$C_6$-Alkenyloxy-, $C_3$-$C_6$-Alkinyloxy- oder eine $C_3$-$C_6$-Alkinylgruppe, wobei diese Gruppen ihrerseits ein bis fünf Halogenatome tragen können;
Di-$C_1$-$C_4$-alkylamino, $C_3$-$C_8$-Cycloalkyl, welches ein bis drei $C_1$-$C_4$-Alkylreste tragen kann;
$C_1$-$C_{10}$-Alkoxycarbonyl, $C_1$-$C_4$-Alkylthio;
eine Phenoxygruppe, wobei der aromatische Rest ein bis fünf Halogenatome und/oder ein bis drei der folgenden Reste tragen kann, $C_1$-$C_4$-Alkyl, $C_1$-$C_4$-Halogenalkyl, $C_1$-$C_4$-Halogenalkoxy, $C_1$-$C_4$-Alkoxy, $C_1$-$C_4$-Alkylthio;
eine $C_1$-$C_{10}$-Alkyl- oder Alkoxygruppe, welche ein bis fünf Halogenatome und/oder einen der folgenden Reste tragen kann: $C_1$-$C_4$-Alkoxy, $C_1$-$C_4$-Alkylthio, Phenyl oder Phenoxy, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Halogenalkoxy, $C_1$-$C_4$-Alkylthio;
bedeuten;
oder einen Naphthylrest, welcher jeweils ein bis drei Halogenatome und/oder einen bis drei der folgenden Reste tragen kann: $C_1$-$C_4$-Alkyl oder $C_1$-$C_2$-Halogenalkyl,
sowie umweltverträgliche Salze der Verbindungen I.

Weiterhin betrifft die Erfindung Verfahren zur Herstellung der Verbindungen I sowie ihre Verwendung als Herbizide und Wachstumsregulatoren sowie neue Salicylsäurederivate der Formel II'

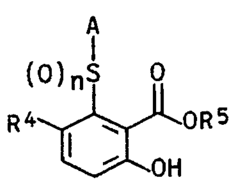

II'

als Zwischenprodukte zur Herstellung der Verbindungen I.

In der Formel II' haben die Reste $R^5$, $R^4$ und A die folgende Bedeutung:

$R^5$
Wasserstoff, ein Alkalimetallkation, das Äquivalent eines Erdalkalimetallkations, ein organisches Ammoniumion;
eine $C_1$-$C_{10}$-Alkylgruppe, welche ein bis fünf Halogenatome und/oder einen der folgenden Reste tragen kann: $C_1$-$C_4$-Alkoxy, $C_1$-$C_4$-Alkylthio, Phenyl, Phenoxy, wobei die Phenylreste jeweils ein bis fünf Halogenatome und/oder ein bis drei der folgenden Reste können: $C_1$-$C_4$-Alkyl, $C_1$-$C_4$-Halogenalkyl, $C_1$-$C_4$-Alkoxy, $C_1$-$C_4$-Halogenalkoxy und/der $C_1$-$C_4$-Alkylthio;

$R^4$
Wasserstoff oder $C_1$-$C_4$-Alkyl;

A

einen ein- bis dreifach, im Fall von Halogen als Substituent ein bis fünffach substituierten Phenylrest

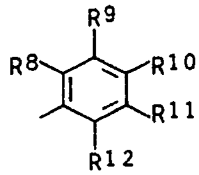

worin $R^8$ - $R^{12}$

Wasserstoff, Halogen, Cyano, Nitro;

eine $C_3$-$C_6$-Alkenyl-, $C_3$-$C_6$-Alkenyloxy-, $C_3$-$C_6$-Alkinyloxy- oder eine $C_3$-$C_6$-Alkinylgruppe, wobei diese Gruppen ihrerseits ein bis fünf Halogenatome tragen können;

Di-$C_1$-$C_4$-alkylamino, $C_3$-$C_8$-Cycloalkyl, welches ein bis drei $C_1$-$C_4$-Alkylreste tragen kann;

$C_1$-$C_{10}$-Alkoxycarbonyl, $C_1$-$C_4$-Alkylthio;

eine Phenoxygruppe, wobei der aromatische Rest ein bis fünf Halogenatome und/oder ein bis drei der folgenden Reste tragen kann, $C_1$-$C_4$-Alkyl, $C_1$-$C_4$-Halogenalkyl, $C_1$-$C_4$-Halogenalkoxy, $C_1$-$C_4$-Alkoxy, $C_1$-$C_4$-Alkylthio;

eine $C_1$-$C_{10}$-Alkyl- oder Alkoxygruppe, welche ein bis fünf Halogenatome und/oder einen der folgenden Reste tragen kann: $C_1$-$C_4$-Alkoxy, $C_1$-$C_4$-Alkylthio, Phenyl oder Phenoxy, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Halogenalkoxy, $C_1$-$C_4$-Alkylthio;

bedeuten;

oder einen Naphthylrest, welcher jeweils ein bis drei Halogenatome und/oder einen bis drei der folgenden Reste tragen kann: $C_1$-$C_4$-Alkyl oder $C_1$-$C_2$-Halogenalkyl,

In der Literatur (EP-A 223 406, EP-A 249 708, EP-A 287 072 und EP-A 287 079) sind herbizid wirksame substituierte Salicylsäuren beschrieben. Ihre Wirkung ist jedoch unbefriedigend.

Aufgabe der vorliegenden Erfindung waren daher neue Salicylsäurederivate mit verbesserten herbiziden Eigenschaften sowie mit pflanzenwachstumsregulierenden Eigenschaften.

Entsprechend dieser Aufgabe wurden die eingangs definierten Verbindungen der Formel I gefunden. Außerdem wurden Verfahren zur Herstellung der Verbindungen I und Verfahren zur Bekämpfung unerwünschten Pflanzenwuchses mit den Verbindungen I gefunden. Es wurde außerdem gefunden, daß Salicylsäurederivate der vorstehend definierten allgemeinen Formel I ausgezeichnete pflanzenwachstumsregulierende Eigenschaften besitzen. Als Zwischenprodukte zur Herstellung der Verbindungen I wurden die neuen Salicylsäurederivate II' gefunden.

Verbindungen der Formel I erhält man beispielsweise, indem man ein entsprechend substituiertes Salicylsäurederivat der Formel II, mit einer entsprechenden Verbindung der Formel III in Gegenwart einer Base umsetzt.

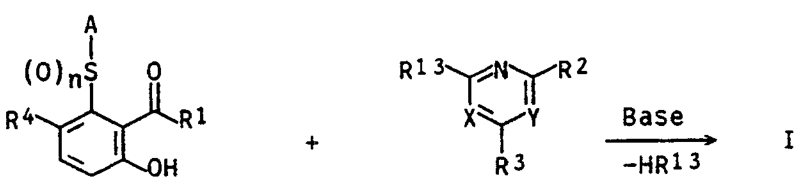


II III

$R^{13}$ in Formel III bedeutet eine übliche nucleofuge Abgangsgruppe, beispielsweise Halogen wie Chlor, Brom und Iod, Aryl- oder Alkylsulfonyl wie Toluolsulfonyl und Methylsulfonyl oder eine andere äquivalente Abgangsgruppe. Verbindungen der Formel III mit einem reaktionsfähigen Substituenten $R^{13}$ sind bekannt z.B. 2,4,6-Trichlorpyrimidin, 2-Alkylthio- oder Arylthio-4,6-dichlorpyrimidin, Cyanursäurechlorid oder mit dem allgemeinen Fachwissen leicht zu erhalten. Als Base können Alkali- oder Erdalkalimetallhydride wie NaH und $CaH_2$, Alkalimetallhydroxide wie NaOH und KOH, Alkalimetallalkohole wie Kalium-tert.-butylat, Alkalimetallcarbonate wie $Na_2CO_3$ und $K_2CO_3$, Alkalimetallamide wie $NaNH_2$ und Lithiumdiisopropylamid oder tertiäre Amine Verwendung finden. Bei Einsatz einer anorganischen Base kann man einen Phasentransferkatalysator zusetzen, wenn dies den Umsatz fördert.

Soweit es sich bei den in der beschriebenen Weise hergestellten Verbindungen der Formel I um

Carbonsäuren handelt (wenn also $R^1$ Hydroxyl bedeutet), können hieraus andere beschriebene Verbindungen z.B. auch dadurch hergestellt werden, daß man die Carbonsäure zunächst auf übliche Weise in eine aktivierte Form wie ein Halogenid oder Imidazolid überführt und dieses dann mit der entsprechenden Hydroxylverbindung umsetzt. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Die Zwischenprodukte der Formel II lassen sich, wenn n für Null und $R^1$ für einen niederen Alkoxirest steht, gemäß dem untenstehenden Schema aus dem 2-Chlormalonat der Formel IV und gegebenfenalls substituiertem Thiophenol HS-A in Gegenwart einer anorganischen oder organischen Base und anschließende thermische Cyclisierung bei Temperaturen von 100 bis 350 °C herstellen.

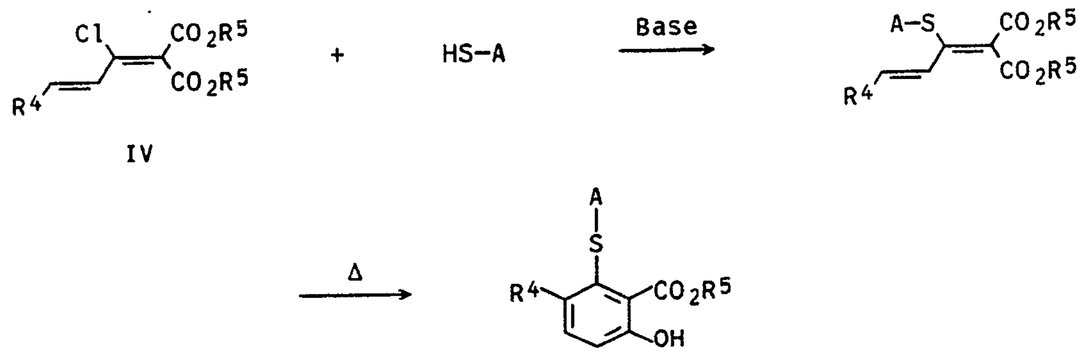


IV

II (n = 0, $R^5$ = Alkyl)

Der als Ausgangsstoff verwendete 2-Chlormalonat IV ist leicht zugänglich, z.B. durch Umsetzung entsprechender Acylmalonate mit Chlorierungsmittel wie Phosphoroxychlorid in Gegenwart von Tributylamin wie in Journal of Organic Chemistry 53, Seite 881, 1988 beschrieben.

Die wie oben beschrieben hergestellten Zwischenprodukte der Formel II (n = 0) fallen üblicherweise als Alkylester an. Diese lassen sich nach bekannten Verfahren zu den Carbonsäuren hydrolysieren. Man kann letztere nun nach literaturbekannten Verfahren in verschiedene Ester überführen, die man zur Darstellung von Wirkstoffen der Formel I gemäß Anspruch 1 benötigt. In entsprechender Weise gelangt man zu den erfindungsgemäßen Zwischenprodukten der Formel II'.

Zur Herstellung der Wirkstoffe der Formel I mit n in der Bedeutung 1 oder 2 gemäß Anspruch 1 kann man die Wirkstoffe der Formel I mit n in der Bedeutung Null mit geeigneten Oxidationsmitteln zum Sulfoxid- oder Sulfon-Derivat. Wahlweise kann man auch die Zwischendprodukte der Formel II (n = 0) mit geeigneten Oxidationsmitteln oxidieren. Als Oxidationsmittel kommen Persäuren z.B. m-Chlorperbenzoesäure, Peressigsäure oder Wasserstoffperoxid in Frage.

Im Hinblick auf die herbizide Wirksamkeit sind Verbindungen I bevorzugt, in denen die Substituenten folgende Bedeutung haben:

$R^1$

Succinyliminooxy,

5-gliedriges Heteroaryl wie Pyrrolyl, Pyrazolyl, Imidazolyl und Triazolyl, insbesondere Imidazolyl und Pyrazolyl, wobei der aromatische Rest über Stickstoff gebunden ist und seinerseits ein bis vier Halogenatome wie vorstehend genannt, insbesondere Fluor und Chlor und/oder ein bis zwei der folgenden Reste tragen kann:

Alkyl, wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl,

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl;

Alkoxy wie vorstehend genannt, mit ein bis vier Kohlenstoffatomen, Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und/oder

Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;
ein Rest $OR^5$, worin

$R^5$

Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion;
Alkyl wie insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, welches ein bis fünf der vorstehend genannten Halogenatome, insbesondere Fluor und Chlor und/oder einen der folgenden Reste tragen kann:
Cyano, Alkoxy bzw. Alkylthio mit ein bis vier Kohlenstoffatomen wie vorstehend genannt, insbesondere Methoxy, Ethoxy, 1-Methylethoxy und Methylthio;
Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;
Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, n-Pentyloxycarbonyl, 1-Methylbutyloxycarbonyl, 2-Methylbutyloxycarbonyl, 3-Methylbutyloxycarbonyl, 1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methylpentyloxycarbonyl, 2-Methylpentyloxycarbonyl, 3-Methylpentyloxycarbonyl, 4-Methylpentyloxycarbonyl, 1,2-Dimethylbutyloxycarbonyl, 1,3-Dimethylbutyloxycarbonyl, 2,3-Dimethylbutyloxycarbonyl, 1,1-Dimethylbutyloxycarbonyl, 2,2-Dimethylbutyloxycarbonyl, 3,3-Dimethylbutyloxycarbonyl, 1,1,2-Trimethylpropyloxycarbonyl, 1,2,2-Trimethylpropyloxycarbonyl, 1-Ethylbutyloxycarbonyl, 2-Ethylbutyloxycarbonyl, 1-Ethyl-2-methylpropyloxycarbonyl, n-Heptyloxycarbonyl, 1-Methylhexyloxycarbonyl, 2-Methylhexyloxycarbonyl, 3-Methylhexyloxycarbonyl, 4-Methylhexyloxycarbonyl, 5-Methylhexyloxycarbonyl, 1-Ethylpentyloxycarbonyl, 2-Ethylpentyloxycarbonyl, 1-Propylbutyloxycarbonyl und Octyloxycarbonyl, insbesondere Methoxycarbonyl, Ethoxycarbonyl, 1-Methylethoxycarbonyl und 1-Methylpropyloxycarbonyl.

Phenyl, Phenoxy oder Phenylcarbonyl, wobei diese aromatischen Reste ihrerseits ein bis fünf Halogenatome wie vorstehend genannt, insbesondere Fluor, Chlor und Brom und/oder ein bis drei der folgenden Reste tragen können:
Alkyl, Halogenalkyl, Alkoxy, Halogenalkoxy und/oder Alkylthio mit jeweils ein bis vier Kohlenstoffatomen wie im allgemeinen und im besonderen vorstehend genannt, oder
$C_1$-$C_{10}$-Alkyl wie vorstehend genannt, welches ein bis fünf Halogenatome wie Fluor, Chlor, Brom oder lod, insbesondere Fluor oder Chlor tragen kann und zusätzlich einen der folgenden Reste trägt:
5-gliedriges Heteroaryl mit ein bis drei Stickstoffatomen wie vorstehend für $R^1$ genannt.

$C_2$-$C_6$-Alkyl, insbesondere $C_2$-$C_4$-Alkyl, das in 2-Stellung substituiert ist durch $C_1$-$C_6$-Alkoxyimino, z.B. Methoxy-, Ethoxy- oder Propoxyimino; $C_3$-$C_6$-Alkenyloxyimino wie 2-Propenyloxyimino, 2-Butenyloxyimino, 3-Butenyloxyimino; $C_3$-$C_6$-Halogenalkenyloxyimino wie 3,3-Dichlor-2-propenyloxyimino, 2,3,3-Trichlor-2-propenoxyimino oder Benzyloxyimino. Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;

Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl, wobei diese Alkenyl- und Alkinylgruppen ein bis fünf der vorstehend im allgemeinen und im besonderen genannten Halogenatome tragen können, bedeutet;

$C_3$-$C_{12}$-Cycloalkyl, insbesondere $C_3$-$C_6$-Cycloalkyl wie Cyclopropyl, Cyclobutyl, Cyclopentyl, Cyclohexyl, das gegebenenfalls durch ein bis drei $C_1$-$C_4$-Alkylreste substituiert ist;

Phenyl, Phenyl ein bis dreifach substituiert durch $C_1$-$C_4$-Alkyl, $C_1$-$C_4$-Halogenalkyl, $C_1$-$C_4$-Alkoxy oder $C_1$-$C_4$-Halogenalkoxy wie Methyl, Ethyl, Propyl, Butyl, Methoxy, Ethoxy, Trifluormethyl, Chlordifluormethyl, Trifluormethoxy, Pentafluorethoxy und Trichlormethoxy oder Phenyl substituiert durch ein bis fünf Halogenatome, z.B. Chlor oder Fluor;

ein Rest $ON=CR^6R^7$, worin

$R^6$ $R^7$

unverzweigtes oder verzweigtes $C_1$-$C_{20}$-Alkyl, vorzugsweise $C_1$-$C_{15}$-Alkyl, insbesondere $C_1$-$C_9$-Alkyl, welches einen Phenyl, einen $C_1$-$C_4$-Alkoxy oder einen $C_1$-$C_4$-Alkylthiorest tragen kann, Phenyl oder gemeinsam $C_3$-$C_{12}$-Alkylen, vorzugsweise $C_4$-$C_7$-Alkylen, welches ein bis drei $C_1$-$C_3$-Alkylgruppen, vorzugsweise Methyl- oder Ethylgruppen tragen kann, bedeutet;

$R^2$, $R^3$

im allgemeinen und im besonderen die bei $R^1$ genannten Alkylgruppen, Halogen, Halogenalkylgruppen, Alkoxygruppen, Halogenalkoxygruppen und/oder Alkylthiogruppen mit jeweils ein bis vier Kohlenstoffatomen sowie Mono- oder Di-$C_1$-$C_4$-alkylamino wie Methylamino, Ethylamino, Propylamino, Isopropylamino, Butylamino oder wie für $R^8$ bis $R^{12}$ vorstehend genannt;

X, Y

Stickstoff oder eine Methingruppe =CH-;

$R^4$

Wasserstoff;

Alkyl mit jeweils ein bis vier Kohlenstoffatomen, insbesondere ein bis drei Kohlenstoffatomen. Beispielsweise seien Methyl, Ethyl, n-Propyl, iso-Propyl genannt.

A

unsubstituiertes oder substituiertes Phenyl, worin als Substituenten $R^8$ bis $R^{12}$ in Betracht kommen: Halogen wie Fluor, Chlor, Brom oder Iod; Cyano; Nitro, ggf. halogensubstituiertes Alkenyl, Alkenyloxy, Alkinyloxy oder Alkinyl mit jeweils 3 bis 6 Kohlenstoffatomen; Di-$C_1$-$C_4$-alkylamino wie Di-Methylamino, Di-Ethylamino, Di-Propylamino, Di-1-Methylethylamino, Di-Butylamino, Di-1-Methylpropylamino, Di-2-Methylpropylamino, Di-1,1-Dimethylethylamino, Ethylmethylamino, Propylmethylamino, 1-Methylethylmethylamino oder Butylmethylamino; gegebenenfalls alkylsubstituiertes Cycloalkyl wie vorstehend für $R^5$ aufgeführt, Alkoxycarbonyl oder Alkylthio wie vorstehend für $R^5$ aufgeführt, gegebenenfalls substituiertes Phenoxy wie unter $R^5$ aufgeführt, $C_1$-$C_{10}$-Alkyl- oder Alkoxy, insbesondere $C_1$-$C_6$, bevorzugt $C_1$-$C_4$-Alkyl oder Alkoxy welche unsubstituiert oder durch die genannten Reste substituiert sind; beispielsweise seien die folgenden substituierten Phenylreste für A genannt:

2-Fluorphenyl, 3-Fluorphenyl, 4-Fluorphenyl, 2,6-Difluorphenyl, 2,4-Diflurophenyl, 2-Fluor-4-trifluormethylphenyl, 2,3-Difluorphenyl, 2-Chlorphenyl, 3-Chlorphenyl, 2-Iodphenyl, 2-Bromphenyl, 2-Chlor-6-Fluorphenyl, Pentafluorphenyl, Pentachlorphenyl, 2,4-Dichlorphenyl, 2,6-Dichlorphenyl, 2-Chlor-4-fluorphenyl, 3,5-Dichlorphenyl, 2-Chlor-6-methylphenyl, 2,3,5-Trichlorphenyl, 2-Methylphenyl, 3-Methylphenyl, 4-Methylphenyl, 2,6-Dimethylphenyl, 2,4-Dimethylphenyl, 3,5-Dimethylphenyl, 2,4,6-Trimethylphenyl, 2-Chlor-4-methylphenyl, 2-Methoxyphenyl, 4-Methoxyphenyl, 4-Chlor-2-methoxyphenyl, 2-Trifluormethylphenyl, 2,3-Dimethyl-4-methoxyphenyl, 4-Dimethylamino-2-methylphenyl, 3-Cyanophenyl, 3-Nitrophenyl, 3-Phenoxyphenyl, 3-(3-Trifluormethylphenoxy)phenyl, 3-Trifluormethylphenyl,

Gegebenenfalls substituiertes Naphthyl für A ist beispielsweise 1-Naphthyl oder 2-Napthyl.

Insbesondere bevorzugt sind Verbindungen der Formel I, in denen

$R^2$

Methoxi, Methyl, Difluormethoxi oder Chlor;

$R^3$
Methoxi, Methyl, Difluormethoxi oder Chlor;

$R^4$
Wasserstoff oder Methyl;

bedeuten und in denen X Stickstoff und Y eine Methingruppe darstellen und $R^1$ und A die im Anspruch genannte Bedeutung haben.

Als Salze der Verbindungen der Formel I kommen umweltverträgliche Salze, beispielsweise Alkalimetallsalze, insbesondere das Kalium- oder oder Natriumsalz, Erdalkalimetallsalze, insbesondere das Calcium-, Magnesium-oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Beispiel 1

2-(4,6-Dimethoxypyrimidin-2-yl)oxi-6-phenylthiobenzoesäure (Verbindung Nr. 1.002)
a) 3-Phenylthio-2-ethoxicarbonyl-2,4-hexadiencarbonsäureethylester
Zu einer Lösung von 25,0 g (0,1 mol) 3-Chlor-2-ethoxicarbonyl-2,4-hexadiencarbonsäureethylester und 13,5 g (0,12 mol) Thiophenol in 75 ml Chloroform werden unter Stickstoff (Ausschluß von Sauerstoff) 12 g (0,12 mol) Triethylamin zugetropft und dann bei Rückfluß 12 Stunden gerührt. Nach dem Waschen, Trocknen und Verdampfen des Lösungsmittels erhält man die obige Verbindung als Rohprodukt (27,5 g Öl)
b) 6-Phenylthio-salicylsäureethylester
27,5 g des obigen Öls wurden in einer Destillationsapparatur bei 240-250 °C Badtemperatur unter Rühren erhitzt bis die Abspaltung von Ethanol (ca. 5 ml Destillat) beendet war. Der abgekühlte Rückstand wurde in 10 % NaOH gelöst und einmal mit Äther extrahiert. Nach Ansäuern der wäßrigen Phase mit konz. HCl, Extraktion des Produktes mit Ether, Trocknen und Verdampfen des Lösungsmittels isolierte man 15 g (55 %) des obigen Esters ($Kp_{10}$: 200 °C; Fp: 48-50 °C).
c) 6-Phenylthio-salicylsäure
0,85 g (3,1 mmol) 6-Phenylthio-salicylsäureethylester werden in eine Lösung von 0,45 g (6,8 mmol) 85 % Kaliumhydroxid in 5 ml Wasser eine Stunde am Rückfluß gekocht. Nach dem Abkühlen wird das Reaktionsgemisch mit Orthophosphorsäure auf pH 3 angesäuert. Nach Absaugen und Trocknen der Kristalle erhält man 0,7 g (91 %) des Produktes vom Schmelzpunkt 150° (Zers.).
d) 2-(4,6-Dimethoxypyrimidin-2-yl)-oxi-6-phenylthio-benzoesäure
Zu einer Lösung von 0,6 g (2,4 mmol) 6-Phenylthio-salicylsäure in 8 ml Dimethylsulfoxid gibt man portionsweise 0,55 g (4,9 mmol) Kalium-tert.-butylat, wobei die Temperatur bis auf 35 °C ansteigt. Nach dem Abkühlen auf Raumtemperatur gibt man 0,53 g (2,4 mmol) 4,6 Dimethoxy-2-methylsulfonylpyrimidin zu und rührt 1 h bei Raumtemperatur nach. Der Kolbeninhalt wird auf eine Mischung von 100 ml kaltem Wasser, 1 ml Orthophosphorsäure und 50 ml Ether gegeben. Die Etherphase wird mit 5 %iger Phosphorsäure und Wasser gewaschen, getrocknet und eingeengt. Nach dem Verrühren des Rohproduktes mit kaltem Toluol isoliert man 0,50 g (54 %) des obigen Produktes vom Schmelzpunkt 135-136 °C.

Beispiel 2

Allgemeine Vorschrift zur Herstellung aromatischer Carbonsäureoximester oder ähnlicher Verbindungen der Formel I:
3,2 mmol der jeweiligen aromatischen 2-(4,6-Dimethoxypyrimidin-2-yl)oxycarbonsäure werden in 20 ml Dimethoxyethan vorgelegt und mit 3,2 mmol Natriumhydrid versetzt, wobei sofort eine Gasentwicklung auftritt. Es wird 1 h bei Raumtemperatur nachgerührt, auf 0 °C gekühlt und mit 3,5 mmol Oxalylchlorid versetzt. Man rührt bei 0 °C 1 h nach und verdampft dann zur Entfernung des überschüssigen Oxalylchlorids etwa 30 % des Lösungsmittel im Vakuum. Man gibt nun 4,2 mmol des jeweiligen Oxims oder einer vergleichbaren Hydroxy-Verbindung gelöst in 10 ml Dimethoxyethan und anschließend 3,2 mmol Pyridin bei 0 °C zu und erwärmt innerhalb von 1 h auf Raumtemperatur. Die Mischung wird in 120 ml kaltes Wasser gegossen und mit Methylenchlorid extrahiert. Die organische Phase wird über Natriumsulfat getrocknet und im Vakuum eingeengt. Die zurückbleibende Substanz kann durch Chromatographie an Silica-Gel weiter

gereinigt werden.

Tabelle 1: Salicylsäurederivate der Formel I (mit X = N und $R^2$ = $OCH_3$)

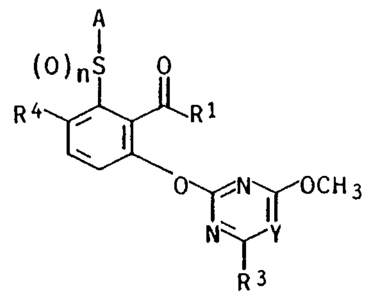

I

| Nr. | $R^1$ | $R^3$ | $R^4$ | A | n | Y | phys.Dat. Fp. [°C] |
|---|---|---|---|---|---|---|---|
| 1.001 | OH | $OCH_3$ | H | Phenyl | 0 | N | |
| 1.002 | OH | $OCH_3$ | H | Phenyl | 0 | CH | 135-136 |
| 1.003 | $OCH_3$ | $OCH_3$ | H | Phenyl | 0 | CH | |
| 1.004 | $OC_2H_5$ | $OCH_3$ | H | Phenyl | 0 | CH | |
| 1.005 | 2-Propeniminoxy | $OCH_3$ | H | Phenyl | 0 | CH | |
| 1.006 | Methylthiomethyloxi | $OCH_3$ | H | Phenyl | 0 | CH | |
| 1.007 | Ethoxycarbonylmethyloxi | $OCH_3$ | H | Phenyl | 0 | CH | |
| 1.008 | Propargyloxi | $OCH_3$ | H | Phenyl | 0 | CH | |
| 1.009 | OH | $OCH_3$ | H | 2-Fluorphenyl | 0 | CH | |
| 1.010 | OH | $OCH_3$ | H | 3-Fluorphenyl | 0 | CH | |
| 1.011 | OH | $OCH_3$ | H | 4-Fluorphenyl | 0 | CH | 240 |
| 1.012 | OH | $OCH_3$ | H | 2-Chlorphenyl | 0 | CH | |
| 1.013 | OH | $OCH_3$ | H | 3-Chlorphenyl | 0 | CH | 142-144 |

Tabelle 1 (Fortsetzung)

| Nr. | $R^1$ | $R^3$ | $R^4$ | A | n | Y | phys.Dat. Fp. [°C] |
|---|---|---|---|---|---|---|---|
| 1.014 | OH | $OCH_3$ | H | 4-Chlorphenyl | 0 | CH | 160 |
| 1.015 | OH | $OCH_3$ | H | 2,4-Dichlorphenyl | 0 | CH | |
| 1.016 | OH | $OCH_3$ | H | 2,5-Dichlorphenyl | 0 | CH | |
| 1.017 | OH | $OCH_3$ | H | 2,4,5-Trichlorphenyl | 0 | CH | |
| 1.018 | OH | $OCH_3$ | H | 2,3,4-Trichlorphenyl | 0 | CH | |
| 1.019 | OH | $OCH_3$ | H | 2-Methylphenyl | 0 | CH | |
| 1.020 | OH | $OCH_3$ | H | 3-Methylphenyl | 0 | CH | 152 |
| 1.021 | OH | $OCH_3$ | H | 4-Methylphenyl | 0 | CH | 163-165 |
| 1.022 | OH | $OCH_3$ | H | 2,4-Dimethylphenyl | 0 | CH | |
| 1.023 | OH | $OCH_3$ | H | 2-Isopropylphenyl | 0 | CH | |
| 1.024 | OH | $OCH_3$ | H | 4-tert.Butylphenyl | 0 | CH | |
| 1.025 | OH | $OCH_3$ | H | 2-Methoxyphenyl | 0 | CH | |
| 1.026 | OH | $OCH_3$ | H | 3-Methoxyphenyl | 0 | CH | |
| 1.027 | OH | $OCH_3$ | H | 4-Methoxyphenyl | 0 | CH | |
| 1.028 | OH | $OCH_3$ | H | 4-Phenoxyphenyl | 0 | CH | |
| 1.029 | OH | $OCH_3$ | H | 4-Bromphenyl | 0 | CH | |
| 1.030 | OH | $OCH_3$ | H | 3-Trifluormethylphenyl | 0 | CH | |
| 1.031 | OH | $OCH_3$ | H | 3-Nitrophenyl | 0 | CH | |
| 1.032 | OH | $OCH_3$ | H | 4-Nitrophenyl | 0 | CH | |
| 1.033 | OH | $OCH_3$ | H | Naphth-1-yl | 0 | CH | |
| 1.034 | OH | $OCH_3$ | H | Naphth-2-yl | 0 | CH | |
| 1.035 | OH | $OCH_3$ | H | Phenyl | 1 | CH | |
| 1.036 | $OC_2H_5$ | $OCH_3$ | H | Phenyl | 1 | CH | |
| 1.037 | OH | $OCH_3$ | H | Phenyl | 2 | CH | 168-170 |
| 1.038 | $OC_2H_5$ | $OCH_3$ | H | Phenyl | 2 | CH | |

Tabelle 2: Salicylsäurederivate der Formel II'

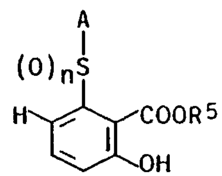

II'

| Nr. | $R^5$ | A | n | phys.Dat. Fp. [°C] |
|---|---|---|---|---|
| 2.001 | H | Phenyl | 0 | 150 (Z) |
| 2.002 | $C_2H_5$ | Phenyl | 0 | 48 - 50 |
| 2.003 | H | 2-Fluorphenyl | 0 | |
| 2.004 | H | 3-Fluorphenyl | 0 | |
| 2.005 | H | 4-Fluorphenyl | 0 | |
| 2.006 | H | 2-Chlorphenyl | 0 | |
| 2.007 | H | 3-Chlorphenyl | 0 | |
| 2.008 | H | 4-Chlorphenyl | 0 | |
| 2.009 | H | 2,4-Dichlorphenyl | 0 | |
| 2.010 | H | 2,5-Dichlorphenyl | 0 | |
| 2.011 | H | 2,4,5-Trichlorphenyl | 0 | |
| 2.012 | H | 2,3,4-Trichlorphenyl | 0 | |
| 2.013 | H | 2-Methylphenyl | 0 | |
| 2.014 | H | 3-Methylphenyl | 0 | |
| 2.015 | H | 4-Methylphenyl | 0 | |
| 2.016 | H | 2,4-Dimethylphenyl | 0 | |
| 2.017 | H | 2-Isopropylphenyl | 0 | |
| 2.018 | H | 4-tert.Butylphenyl | 0 | |
| 2.019 | H | 2-Methoxyphenyl | 0 | |

Tabelle 2 (Fortsetzung)

| Nr. | $R^5$ | A | n | phys.Dat. Fp. [°C] |
|---|---|---|---|---|
| 2.020 | H | 3-Methoxyphenyl | 0 | |
| 2.021 | H | 4-Methoxyphenyl | 0 | |
| 2.022 | H | 4-Phenoxyphenyl | 0 | |
| 2.023 | H | 4-Bromphenyl | 0 | |
| 2.024 | H | 3-Trifluormethylphenyl | 0 | |
| 2.025 | H | 3-Nitrophenyl | 0 | |
| 2.026 | H | 4-Nitrophenyl | 0 | |
| 2.027 | H | Naphth-1-yl | 0 | |
| 2.028 | H | Naphth-2-yl | 0 | |
| 2.029 | H | Phenyl | 1 | |
| 2.030 | $C_2H_5$ | Phenyl | 1 | |
| 2.031 | H | Phenyl | 2 | |
| 2.032 | $C_2H_5$ | Phenyl | 2 | |

Die erfindungsgemäßen herbiziden und wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.002 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 1.002 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.002 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.002 werden in einer Mischung gelöst, die aus 25 Gewichtsteilen Cyclohexanon, 65 Gewichtsteilen einer Mineralölfraktion vom Siedepunkt 210 bis 280 °C und 10

Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.002 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.002 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.002 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.002 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. Man vermischt 90 Gewichtsteile der Verbindung Nr. 1.002 mit 10 Gewichtsteilen N-Methyl-α-pyrrolidon und erhält eine Lösung, die zur Anwendung in Form kleinster Tropfen geeignet ist.

X. 20 Gewichtsteile der Verbindung Nr. 1.002 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäureund 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

XI. 20 Gewichtsteile der Verbindung Nr. 1.002 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XII. 20 Gewichtsteile des Wirkstoffs Nr. 1.002 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XIII. 20 Gewichtsteile des Wirkstoffs Nr. 1.002 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

XIV. 3 Gewichtsteile des Wirkstoffs Nr. 1.002 werden mit 97 Gewichtsteilen feinteiligem Kaolin vermischt. Man erhält auf diese Weise ein Stäubemittel, das 3 Gew.% des Wirkstoffs enthält.

XV. 30 Gewichtsteile des Wirkstoffs Nr. 1.002 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

XVI. 20 Gewichtsteile des Wirkstoffs Nr. 1.002 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenolsulfonsäure-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit, Zielpflanzen und Wachstumsstadium 0,001 bis 3,0, vorzugsweise 0,005 bis 0,5 kg/ha aktive Substanz (a.S.).

Die wachstumsregulierend wirkenden Salicylsäurederivate der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel Ia im Pflanzenanbau, in der Landwirtschaft und im Gartenbau, werden eingige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzender Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Salicylsäurederivate der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässertwerden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Plfanzenverträglichkeit kann die Aufwandmenge stark variiert werden.

Bei der Saatgutbehandlung werden im allgemeinen Wirkstoffmengen von 0,001 bis 50 g, vorzugsweise 0,01 bis 10 g, je Kilogramm Saatgut, benötigt.

Für die Blatt- und Bodenbehandlung sind im allgemeinen Gaben von 0,001 bis 10 kg/ha, bevorzugt 0,01 bis 3 kg/ha, insbesondere 0,01 bis 0,5 kg/ha als ausreichend zu betrachten.

Die Applikation der herbiziden Mittel bzw. der Wirkstoffe kann im Vorauflauf-oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,005 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |

| Botanischer Name | Deutscher Name |
|---|---|
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohne |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Anwendungsbeispiele

Die herbizide Wirkung der Salicylsäurederivate I ließ sich durch Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies

nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,125 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Abkürzung | Deutscher Name |
|---|---|---|
| Chenopodium album | CHEAL | Weißer Gänsefuß |
| Amaranthus retroflexus | AMARE | Zurückgekrümmter Fuchsschwanz |
| Sesbania exaltata | SEBEX | Turibaum |

Mit 0,125 kg/ha a.S. im Nachauflaufverfahren eingesetzt, lassen sich mit der Beispielsverbindung Nr. 1.002 breitblättrige unerwünschte Pflanzen sehr gut bekämpfen.

Die wachstumsregulierende Wirkung der Salicylsäurederivate I ist den Beispielen A und B zu entnehmen.

Beispiel A

Untersuchung auf wachstumsregulierende Wirkung der Verbindung Nr. 1.002 in einem Prüfsystem mit Wasserlinsen (Lemna pauciostata)

Die Pflanzen wurden photomixotroph (Zusatz von 1 % Saccharose im anorganischen Nährmedium) unter sterilen Bedingungen im Dauerlicht angezogen. Die Prüfsubstanzen wurden in Aceton gelöst und in Aufwandmengen von $10^{-7}$ bis $10^{-10}$ mol/l den Wasserlinsen zugegeben. Nach 8 Tagen wurde die Frischgewichtszunahme der Pflanzen bestimmt und die wachstumsregulierende Wirkung der o.g. Verbindung in % Hemmung des Wachstums zur Kontrolle verrechnet (0 = keine Hemmung, 100 = totale Hemmung des Wachstums).

| Aufwandmenge (mol x $l^{-1}$) | Hemmung des Wachstums |
|---|---|
| $10^{-7}$ | 95 |
| $10^{-8}$ | 89 |
| $10^{-9}$ | 5 |
| $10^{-10}$ | 0 |

Beispiel B

Zur Bestimmung der wachstumsregulierenden Eigenschaft der Verbindung Nr. 1.002 wurden die Testpflanzen auf ausreichend mit Nährstoffen versorgtem Kultursubstrat in Kunststoffgefäßen von ca. 12,5 cm Durchmesser angezogen.

Im Nachauflaufverfahren wurde die zu prüfende Substanz in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen B.1 und B.2 zu entnehmen.

Tabelle B.1

Sommergerste, "Aramir"

Nachauflauf-Blattbehandlung

| Wirkstoff-Nr. | Konzentration mg Wirkstoff/Gefäß | relative Wuchshöhen |
|---|---|---|
| unbehandelt | - | 100 |
| 1.002 | 0,0063 | 103,0 |
| | 0,0125 | 99,7 |
| | 0,05 | 88,0 |
| | 0,1 | 78,1 |
| | 0,4 | 56,5 |

Tabelle B.2

Sommerweizen, "Ralle"

Nachauflauf-Blattbehandlung

| Wirkstoff-Nr. | Konzentration mg Wirkstoff/Gefäß | relative Wuchshöhen |
|---|---|---|
| unbehandelt | - | 100 |
| 1.002 | 0,0063 | 99,0 |
| | 0,0125 | 95,8 |
| | 0,05 | 92,7 |
| | 0,1 | 76,7 |
| | 0,4 | 54,3 |

## Ansprüche

1. Salicylsäurederivate der Formel I,

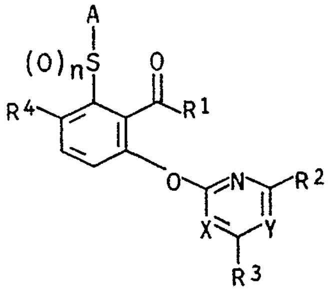

I

in der die Substituenten folgende Bedeutung haben:

$R^1$

eine Succinyliminooxygruppe;

ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

einen Rest -$OR^5$, worin

$R^5$

Wasserstoff, ein Alkalimetallkation, das Äquivalent eines Erdalkalimetallkations oder ein organisches Ammoniumion;

eine $C_3$-$C_{12}$-Cycloalkylgruppe, welche ein bis drei $C_1$-$C_4$-Alkylreste tragen kann;

eine $C_1$-$C_{10}$-Alkylgruppe, welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, $C_3$-$C_{12}$-Cycloalkyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

eine $C_1$-$C_{10}$-Alkylgruppe, welche ein bis fünf Halogenatome tragen kann und einen der folgenden Reste trägt: ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

eine $C_2$-$C_6$-Alkylgruppe, welche in der 2-Position einen der folgenden Reste trägt: $C_1$-$C_6$-Alkoxyimino, $C_3$-$C_6$-Alkenyloxyimino, $C_3$-$C_6$-Halogenalkenyloxyimino oder Benzyloxyimino;

eine $C_3$-$C_6$-Alkenyl- oder eine $C_3$-$C_6$-Alkinylgruppe, wobei diese Gruppen ihrerseits ein bis fünf Halogenatome tragen können;

unsubstituiertes oder ein- bis dreifach durch $C_1$-$C_4$-Alkyl, $C_1$-$C_4$-Halogenalkyl, $C_1$-$C_4$-Alkoxy oder $C_1$-$C_4$-Halogenalkoxy substituiertes oder ein- bis fünffach durch Halogen substituiertes Phenyl;

bedeutet, oder

einen Rest $ON=CR^6R^7$, worin

$R^6$ und $R^7$

$C_1$-$C_{20}$-Alkyl, welches einen Phenylrest, eine $C_1$-$C_4$-Alkoxy- und/oder eine $C_1$-$C_4$-Alkylthiogruppe tragen kann, Phenyl oder gemeinsam eine $C_3$-$C_{12}$-Alkylenkette, welche ein bis drei $C_1$-$C_3$-Alkylgruppen tragen kann

bedeuten;

$R^2$, $R^3$

$C_1$-$C_4$-Alkyl, Halogen, $C_1$-$C_4$-Halogenalkyl, $C_1$-$C_4$-Alkoxy, $C_1$-$C_4$-Halogenalkoxy, $C_1$-$C_4$-Alkylthio, Mono- oder Di-$C_1$-$C_4$-alkylamino;

n

Null, 1 oder 2

X,Y

ein Stickstoffatom oder eine Methingruppe =CH-;

$R^4$

Wasserstoff oder $C_1$-$C_4$-Alkyl;

A

einen gegebenenfalls ein bis dreifach substituierten, im Fall vor Halogen als Substituent ein bis fünffach substituierten Phenylrest

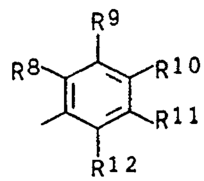

worin $R^8$ - $R^{12}$

Wasserstoff, Halogen, Cyano, Nitro;

eine $C_3$-$C_6$-Alkenyl-, $C_3$-$C_6$-Alkenyloxy-, $C_3$-$C_6$-Alkinyloxy- oder eine $C_3$-$C_6$-Alkinylgruppe, wobei diese Gruppen ihrerseits ein bis fünf Halogenatome tragen können;

Di-$C_1$-$C_4$-alkylamino, $C_3$-$C_8$-Cycloalkyl, welches ein bis drei $C_1$-$C_4$-Alkylreste tragen kann;

$C_1$-$C_{10}$-Alkoxycarbonyl, $C_1$-$C_4$-Alkylthio;

eine Phenoxygruppe, wobei der aromatische Rest ein bis fünf Halogenatome und/oder ein bis drei der folgenden Reste tragen kann, $C_1$-$C_4$-Alkyl, $C_1$-$C_4$-Halogenalkyl, $C_1$-$C_4$-Halogenalkoxy, $C_1$-$C_4$-Alkoxy, $C_1$-$C_4$-Alkylthio;

eine $C_1$-$C_{10}$-Alkyl- oder Alkoxygruppe, welche ein bis fünf Halogenatome und/oder einen der folgenden Reste tragen kann: $C_1$-$C_4$-Alkoxy, $C_1$-$C_4$-Alkylthio, Phenyl oder Phenoxy, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Halogenalkoxy, $C_1$-$C_4$-Alkylthio;

bedeuten;

oder einen Naphthylrest, welcher jeweils ein bis drei Halogenatome und/oder einen bis drei der folgenden Reste tragen kann: $C_1$-$C_4$-Alkyl oder $C_1$-$C_2$-Halogenalkyl,

sowie umweltverträgliche Salze der Verbindungen I.

**2.** Verfahren zur Herstellung der Verbindungen der Formel I gemäß Anspruch 1, dadurch gekennzeichnet, daß man ein entsprechendes Salicylsäurederivat der Formel II

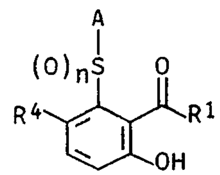

II

in an sich bekannter Weise mit einem Heterocyclus der Formel III,

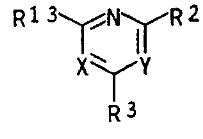

III

worin $R^{13}$ für eine nucleofuge Abgangsgruppe steht, in Gegenwart einer anorganischen oder organischen Base umsetzt.

**3.** Verfahren zur Herstellung der Verbindungen der Formel I gemäß Anspruch. 1, dadurch gekennzeichnet, daß man die freie Salicylsäure der Formel I'

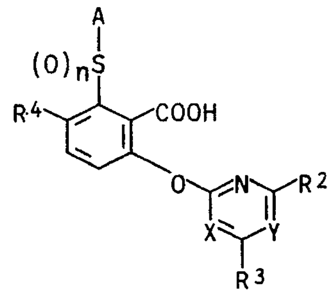

I'

zunächst in an sich bekannter Weise in das Halogenid oder eine andere aktivierte Form der Carbonsäure überführt und diese dann gegebenenfalls in Gegenwart einer anorganischen oder organischen Base mit einem gegebenenfalls substituierten Alkohol, einem Azol, einem Oxim oder N-Hydroxysuccinylimin umsetzt.

4. Herbizides Mittel, enthaltend eine Verbindung der Formel I gemäß Anspruch 1.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

6. Verwendung von Salicylsäurederivaten der Formel I gemäß Anspruch 1 als Herbizide.

7. Mittel zur Beeinflussung des Pflanzenwachstums, enthaltend ein Salicylsäurederivat der Formel I gemäß Anspruch 1.

8. Verfahren zur Regulierung des Pflanzenwachstums, dadurch gekennzeichnet, daß man eine regulativ wirksame Menge eines Salicylsäurederivats der allgemeinen Formel I gemäß Anspruch 1 auf die Samen, die Pflanzen und/oder deren Lebensraum einwirken läßt.

9. Neue Salicylsäurederivate der allgemeinen Formel II'

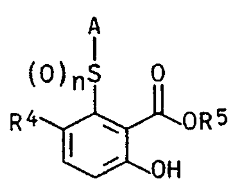

II'

in der die Substituenten folgende Bedeutung haben:

$R^5$
Wasserstoff, ein Alkalimetallkation, das Äquivalent eines Erdalkalimetallkations, ein organisches Ammoniumion;

eine $C_1$-$C_{10}$-Alkylgruppe, welche ein bis fünf Halogenatome und/oder einen der folgenden Reste tragen kann: $C_1$-$C_4$-Alkoxy, $C_1$-$C_4$-Alkylthio, Phenyl, Phenoxy, wobei die Phenylreste jeweils ein bis fünf Halogenatome und/oder ein bis drei der folgenden Reste können: $C_1$-$C_4$-Alkyl, $C_1$-$C_4$-Halogenalkyl, $C_1$-$C_4$-Alkoxy, $C_1$-$C_4$-Halogenalkoxy und/der $C_1$-$C_4$-Alkylthio;

n
Null, 1 oder 2

$R^4$
Wasserstoff oder $C_1$-$C_4$-Alkyl;

A

einen ein- bis dreifach, im Fall von Halogen als Substituent ein bis fünffach substituierten Phenylrest

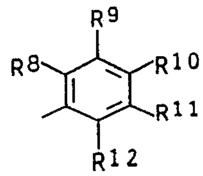

worin $R^8$ - $R^{12}$ Wasserstoff, Halogen, Cyano, Nitro;

eine $C_3$-$C_6$-Alkenyl-, $C_3$-$C_6$-Alkenyloxy-, $C_3$-$C_6$-Alkinyloxy- oder eine $C_3$-$C_6$-Alkinylgruppe, wobei diese Gruppen ihrerseits ein bis fünf Halogenatome tragen können;

Di-$C_1$-$C_4$-alkylamino, $C_3$-$C_8$-Cycloalkyl, welches ein bis drei $C_1$-$C_4$-Alkylreste tragen kann;

$C_1$-$C_{10}$-Alkoxycarbonyl, $C_1$-$C_4$-Alkylthio;

eine Phenoxygruppe, wobei der aromatische Rest ein bis fünf Halogenatome und/oder ein bis drei der folgenden Reste tragen kann, $C_1$-$C_4$-Alkyl, $C_1$-$C_4$-Halogenalkyl, $C_1$-$C_4$-Halogenalkoxy, $C_1$-$C_4$-Alkoxy, $C_1$-$C_4$-Alkylthio;

eine $C_1$-$C_{10}$-Alkyl- oder Alkoxygruppe, welche ein bis fünf Halogenatome und/oder einen der folgenden Reste tragen kann: $C_1$-$C_4$-Alkoxy, $C_1$-$C_4$-Alkylthio, Phenyl oder Phenoxy, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Halogenalkoxy, $C_1$-$C_4$-Alkylthio,

bedeuten;

einen Naphthylrest, welcher jeweils ein bis drei Halogenatome und/oder einen bis drei der folgenden Reste tragen kann: $C_1$-$C_4$-Alkyl oder $C_1$-$C_2$-Halogenalkyl.

**10.** Salicylsäurederivate der Formel I gemäß Anspruch 1, in der $R^2$ $OCH_3$, X Stickstoff, Y die Methingruppe und A sowie die Reste $R^1$, $R^3$ und $R^4$ die in Anspruch 1 genannte Bedeutung haben.